# FASCICULE DE BREVET EUROPEEN

(11) **EP 0 725 118 B1**
(45) Date de publication et mention de la délivrance du brevet: **11.09.1996**
(21) Numéro de dépôt: 95402784.3
(22) Date de dépôt: 11.12.1995
(51) Int. Cl.: C09K 3/14, A61K 7/16

(54) **Compositions à base d'un système abrasif et d'un système tensio-actif**
Schleifmittelzusammensetzung mit Tensidsystem
Composition based on an abrasive and a surfactant system

(30) Priorité: 30.01.1995 FR 9501039
(43) Date de publication de la demande: 07.08.1996
(73) Titulaire: L'OREAL, F-75008 Paris (FR)
(72) Inventeur: Ascione, Jean-Marc, F-75018 Paris (FR); Sterle, Pascal, F-95230 Soisy S/Montmorency (FR)
(74) Mandataire: Andral, Christophe André Louis

(56) Documents cités:
- CH-A- 652 302
- FR-A- 2 546 751
- FR-A- 2 603 800
- GB-A- 2 163 348
- US-A- 4 837 008

## Description

La présente invention concerne de nouvelles compositions comprenant un système abrasif contenant un ou plusieurs bicarbonate(s) de métal alcalin, et un système tensio-actif contenant un ou plusieurs tensio-actif(s) non-ionique(s) poly(hydroxypropyléther) d'une part et un laurylsulfate de métal alcalin, de métal alcalino-terreux ou d'ammonium d'autre part, ces compositions étant notamment représentées par des compositions pour l'hygiène bucco-dentaire.

L'invention concerne également l'utilisation de ces compositions comme, ou pour la fabrication d'un, dentifrice sous la forme d'une pâte, d'un gel, d'un liquide ou d'une gomme à mâcher.

Dans le domaine de l'hygiène bucco-dentaire, et en particulier des dentifrices, on utilise couramment des bicarbonates de métal alcalin, notamment pour neutraliser les acides responsables de la carie, et assurer l'abrasivité.

De plus, pour formuler les dentifrices, on a jusqu'ici utilisé des agents tensio-actifs de type anionique, dont le rôle est d'apporter à la fois un pouvoir moussant et un pouvoir détergent suffisants. Parmi ceux-ci, on peut citer plus particulièrement le lauryl sulfate de sodium, qui est en effet le plus largement utilisé.

Ces dentifrices au laurylsulfate ne sont pas toujours bien tolérés par les couches superficielles de l'épithélium buccal, ce problème étant d'autant plus délicat que l'utilisation des dentifrices, comme chacun sait, est répétitive.
Plusieurs cas d'allergies ont d'ailleurs été rapportés dans la littérature, (BARKVOLL P. ROLLA G. "Possible effects of Sodium Lauryl Sulfate on the oral mucosa", - J. of Dental Research - 1989 - Vol 68/Special issue, Abstract 996, page 991.), et en effet, la demanderesse a observé, à la suite d'expériences de laboratoire, que des formulations, contenant des bicarbonates de métal alcalin et du laurylsulfate notamment de sodium, présentaient une agressivité non négligeable envers la muqueuse buccale.

Dans la demande de brevet français N° 94 14862, la demanderesse a décrit des compositions comprenant, dans un support aqueux, un système abrasif contenant un ou plusieurs bicarbonate(s) de métal alcalin, et un système tensio-actif comprenant un ou plusieurs tensio-actif(s) non-ionique(s) poly(hydroxypropyléther) et leur utilisation dans des compositions à usage bucco-dentaire, en particulier des dentifrices.
De telles compositions présentent pour avantage de posséder une abrasivité très supérieure à celle des compositions antérieurement connues comprenant du bicarbonate de métal alcalin et du laurylsulfate de sodium, tout en conservant un excellent pouvoir moussant et détergent.

A la suite d'expériences complémentaires conduites sur les compositions décrites dans la demande de brevet précitée, en particulier pour évaluer leur tolérance par la muqueuse buccale, la demanderesse a constaté que lesdites compositions étaient en outre moins agressives que celles de l'art antérieur comprenant du bicarbonate de métal alcalin et du laurylsulfate de sodium, mais qu'il serait souhaitable que cette tolérance puisse encore être améliorée.

Et c'est en poursuivant ses recherches que la demanderesse a maintenant découvert, de façon totalement inattendue et surprenante, que l'addition d'un laurylsulfate de métal alcalin, de métal alcalino-terreux ou d'ammonium, à des compositions comprenant un système abrasif contenant un ou plusieurs bicarbonates de métal alcalin et un système tensio-actif comprenant un ou plusieurs tensio-actif(s) non-ioniques ply(hydroxypropyléther), permettait de diminuer considérablement l'agressivité de ces compositions vis à vis de la muqueuse buccale.

Cette découverte est à la base de la présente invention.

La présente invention a ainsi pour objet de nouvelles compositions du type comprenant un système abrasif contenant un ou plusieurs bicarbonate(s) de métal alcalin, et un système tensio-actif contenant un ou plusieurs tensio-actif(s) poly(hydroxypropyléther), qui sont caractérisées par le fait que ledit système tensio-actif comprend en outre au moins un laurylsulfate de métal alcalin, de métal alcalino-terreux ou d'ammonium.

La présente invention a également pour objet des compositions de ce type pour un usage bucco-dentaire, en particulier des dentifrices.

Elle vise également l'utilisation de ces compositions comme, ou pour la fabrication d'un, dentifrice qui peut revêtir la forme d'une pâte, d'un gel, d'un liquide ou d'une gomme à mâcher.

Mais d'autres caractéristiques, aspects, objets et avantages de l'invention apparaîtront encore plus clairement à la lecture de la description et des exemples qui suivent.

Le ou les bicarbonates de métal alcalin qui peuvent être utilisés selon l'invention sont de préférence choisis parmi les bicarbonates de sodium ou de potassium.

Parmi les laurylsulfates, on préfère avantageusement le laurylsulfate de sodium, le laurylsulfate de magnésium et le laurylsulfate d'ammonium, et plus particulièrement encore le laurylsulfate de sodium.

Les tensio-actifs de type poly(hydroxypropyléther) sont des produits déjà connus en soi.

De préférence, selon la présente invention, le ou les tensio-actifs non-ioniques poly(hydroxypropyléther) sont choisis parmi les composés de structures suivantes:
**(A)** Les composés répondant à la formule **(I)**:

   RO-[C₃H₅ (OH)O]ₙ - H (I)

   dans laquelle le groupement -[C₃H₅ (OH)O]- représente les structures (I.a), (I.b), et (I.c) suivantes, prises ensemble ou séparément : 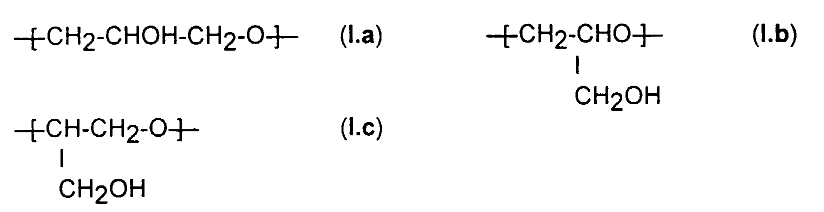 et R et n ont, ensemble, l'une des significations ci-dessous :
   a) R désigne un radical ou un mélange de radicaux alkyles en C₁₀-C₁₄ et n est un nombre entier ou décimal variant de 2 à 10 et de préférence de 3 à 6.
   b) R désigne un groupement de formule **(II)**:

      R₂-CO NH-CH₂-CH₂-O-CH₂-CH₂- (II),

      dans laquelle R₂ désigne un radical ou un mélange de radicaux alkyles et/ou alcényles en C₁₁-C₁₇,
      et n désigne un nombre entier ou décimal variant de 1 à 5 et de préférence de 1,5 à 4.
   c) R désigne un groupement de formule **(III)**:

      R₃-CHOH-CH₂- (III),

      dans laquelle R₃ désigne un radical aliphatique, cycloaliphatique, arylaliphatique, en C₇-C₂₁, et leurs mélanges, les chaînes aliphatiques désignant en particulier des chaînes alkyles pouvant comporter de 1 à 6 groupements éther, thioéther et/ou hydroxyméthylène,
      et n désigne un nombre entier ou décimal variant de 1 à 10.
      Ces tensio-actifs de formule (I) peuvent être préparés selon les procédés décrits dans les brevets FR 1 477 048, 2 328 763, et 2 091 516.
**(B)** Les composés préparés par condensation, en catalyse acide, et à une température allant de 50 à 120°C, de 2 à 10 et de préférence de 2,5 à 6 moles de glycidol par mole d'alcool ou d'alpha-diol comportant 10 à 14 atomes de carbone, le glycidol étant ajouté lentement à l'alcool ou à l'alpha-diol.
   Le procédé de préparation de ces composés est décrit dans le brevet FR 2 169 787.
**(C)** Les composés préparés par polyaddition de monochlorhydrine du glycérol sur un composé organique polyhydroxylé en présence d'une base forte, avec élimination au fur et à mesure de l'eau par distillation.
   Ces composés sont décrits dans le brevet français FR 2 574 786.

Parmi les tensio-actifs non-ioniques poly(hydroxypropyléther) décrits dans les paragraphes (A), (B) et (C) ci-avant, les composés plus particulièrement préférés selon l'invention sont :
i) ceux de formule **(IV)** et **(V)** suivantes : 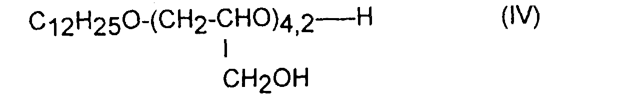 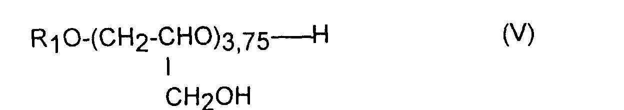 formule (V) dans laquelle R₁ désigne un mélange de radicaux alkyles en C₁₀H₂₁ et C₁₂H₂₅.
2i) les composés préparés par condensation, en catalyse alcaline, de 3,5 moles de glycidol sur un alpha-diol ayant 12 atomes de carbone, selon le procédé décrit dans le brevet FR 2 091 516.
3i) les composés de formule **(VI)** suivante :

   R₂-CONH-CH₂-CH₂-O-CH₂-CH₂-O(̵CH₂-CHOH-CH₂-O)_{3,5}―H (VI)

   dans laquelle R₂ désigne un mélange de radicaux comprenant les radicaux alkyles et alcényles suivants: C₁₁H₂₃, C₁₃H₂₇, les radicaux dérivés des acides gras du coprah et le radical dérivé de l'acide oléique.
4i) les composés préparés par condensation de 3,5 moles de glycidol sur un mélange d'alpha-diols en C₁₁-C₁₄, décrits dans le brevet FR 2 091 516.

Le dodécanediol polyglycérolé à 3,5 moles de glycérol est plus particulièrement préféré.

Dans les compositions selon l'invention, le ou les bicarbonates de métal alcalin sont généralement présents dans des concentrations pondérales comprises entre environ 0,5 et 80% et de préférence entre environ 1 et 50%, le ou les tensio-actifs non-ioniques poly(hydroxypropyléther) sont généralement présents dans des concentrations pondérales comprises entre environ 0,01 et 10% et de préférence entre environ 0,05 et 5%, et le laurylsulfate de métal alcalin, de métal alcalino-terreux ou d'ammonium est généralement présent dans des concentrations pondérales comprises entre environ 0,01 et 10% et de préférence entre environ 0,05 et 5%, toutes ces concentrations pondérales étant exprimées par rapport au poids total de la composition.

Les compositions conformes à l'invention qui sont destinées à l'hygiène bucco-dentaire peuvent contenir, outre le ou les bicarbonate(s) de métal alcalin, le ou les tensio-actif(s) non-ionique(s) poly(hydroxypropyléther) et le ou les laurylsulfates de métal alcalin, de métal alcalino-terreux ou d'ammonium, à titre de simple véhicule (support) ou pour leur activité propre, des excipients ou des ingrédients habituellement utilisés dans les produits à usage bucco-dentaire.

Bien entendu, on veillera à choisir ces composés complémentaires de manière telle que les propriétés avantageuses attachées à l'association ternaire conforme à l'invention ne soient pas, ou substantiellement pas, altérées par l'adjonction envisagée.

Des compositions conformes à l'invention sont préparées selon les procédés usuels correspondant aux véhicules choisis. Le véhicule physiologiquement acceptable peut être de différente nature selon la forme choisie pour la composition : milieu aqueux ou hydroalcoolique épaissi ou non, excipient pâteux ou solide, gomme, etc...

Selon les formes désirées, ces compositions peuvent également contenir d'autres agents abrasifs, parmi lesquels on peut citer par exemple la silice, l'alumine, le phosphate dicalcique et le carbonate de calcium, des agents anticaries comme par exemple des fluorures de sodium ou de potassium ou d'amine, du monofluorophosphate de sodium, des agents antibactériens tels que par exemple la chlorhexidine, l'alexidine, l'héxétidine, le chlorure de cétyl-pyridinium, le 2,4,4'-trichloro 2'-hydroxydiphényléther, des agents anti-inflammatoires, des agents anti-halitoses, des agents antitaches et/ou antitartre, des antiradicaux libres, des anti-oxydants, des enzymes, des vitamines, des oligo-éléments, des agents hémostatiques, des agents cicatrisants et des agents actifs sur la gencive.

Elles peuvent contenir par ailleurs d'autres agents usuels tels que des agents de cohésion, des agents édulcorants, humectants ou rafraîchissants, des agents conservateurs, des colorants, des arômes, des agents de sapidité, des agents peptisants et des agents plastifiants.

Des exemples concrets, mais nullement limitatifs, illustrant l'invention vont maintenant être donnés.

### EXEMPLE 1 :

Suivant le test "in vitro" décrit dans la publication J.A.O.C.S. - 1989 - Vol 66 n°9, intitulée: "An in vitro Method for Evaluation of the irritancy of Anionic Surfactants", par les auteurs A.S. ANAVKAR and C.V. NATRAJ, on a mesuré l'agressivité, vis à vis de la muqueuse buccale, de trois solutions aqueuses définies ci-dessous (S1, S2, S3), le principe du test consistant à extraire, par la solution à étudier, un colorant bleu (Coomassie Brilliant Blue R 250), emprisonné dans une matrice protéique (gélatine), et à quantifier la quantité ainsi extraite de colorant en mesurant par spectrophotométrie l'absorbance de la solution finale.

Plus la quantité de colorant extraite est faible, plus faible est l'absorbance et plus faible est l'agressivité.

Pour chacune des solutions étudiées, les absorbances obtenues, mesurées à 590 nm, ont été les suivantes :

| SOLUTION AQUEUSE contenant : | | | | ABSORBANCE |
|---|---|---|---|---|
| | NaHCO3 | Dodécanediol polyglycérolé à 3,5 moles de glycérol | Laurylsulfate de sodium | |
| S1 | 5% | | 1% | 0,70 |
| S2 | 5% | 1% | | 0,54 |
| S3 | 5% | 1% | 1% | 0,09 |

D'après ces résultats de mesure, la solution aqueuse selon l'invention, contenant 5% de bicarbonate de sodium, 1% de dodécanediol polyglycérolé à 3,5 moles de glycérol et 1% de laurylsulfate de sodium, présente l'absorbance la plus faible et par conséquent l'agressivité la plus faible.

Ces résultats démontrent que l'addition de laurylsulfate de sodium à une solution de bicarbonate de sodium et de dodécanediol polyglycérolé à 3,5 moles de glycérol, diminue de plus de cinq fois l'agressivité de cette solution.

Ces résultats sont d'autant plus surprenants, qu' associé seul au bicarbonate de sodium, le laurylsulfate de sodium présente une agressivité environ huit fois supérieure à la solution conforme à l'invention.

### EXEMPLE 2 :

On illustre ici une pâte dentifrice conforme à l'invention.

| | |
|---|---|
| Bicarbonate de sodium Codex 0/13 vendu par la societé SOLVAY | 25g |
| Silice épaississante, vendue sous la dénomination "SIDENT 22S" par la société DEGUSSA | 9,5g |
| Carboxyméthylcellulose de sodium, vendue sous la dénomination "BLANOSE 9M31F" par la société HERCULES. | 1,25g |
| Sorbitol en solution aqueuse à 70% de matière active (MA) | 15,4g MA |
| Dodécanediol polyglycérolé à 3,5 moles de glycérol | 0,75g |
| Laurylsulfate de sodium | 0,75g |
| Dioxyde de titane | 0,8g |
| Monofluorophosphate de sodium | 0,8g |
| Conservateur, édulcorant, arôme | qs |
| Eau qsq | 100g |

## Revendications

1. Compositions du type comprenant un système abrasif contenant un ou plusieurs bicarbonate(s) de métal alcalin, et un système tensio-actif contenant un ou plusieurs tensio-actif(s) non-ionique(s) poly(hydroxypropyléther), caractérisées par le fait que ledit système tensio-actif comprend en outre au moins un laurylsulfate de métal alcalin, de métal alcalino-terreux ou d'ammonium.

2. Compositions selon la revendication 1, caractérisées par le fait que le ou les bicarbonates de métal alcalin sont choisis parmi le bicarbonate de sodium ou le bicarbonate de potassium.

3. Compositions selon les revendications 1 ou 2, caractérisées par le fait que le laurylsulfate est un laurylsulfate de métal alcalin.

4. Compositions selon la revendication 3, caractérisées par le fait que le laurylsulfate de métal alcalin est un laurylsulfate de sodium.

5. Compositions selon les revendications 1 ou 2, caractérisées par le fait que le laurylsulfate est un laurylsulfate de métal alcalino-terreux.

6. Compositions selon la revendication 5, caractérisées par le fait que le laurylsulfate de métal alcalino-terreux est un laurylsulfate de magnésium.

7. Compositions selon les revendications 1 ou 2, caractérisées par le fait que le laurylsulfate est un laurylsulfate d'ammonium.

8. Compositions selon l'une quelconque des revendications précédentes, caractérisées par le fait que le ou les tensio-actifs non-ioniques poly(hydroxypropyléther) sont choisis parmi les composés suivants :
**(A)** Les composés répondant à la formule **(I)** :
RO⁅C₃H₅ (OH)O]ₙ - H (I)
dans laquelle le groupement -[C₃H₅ (OH)O]- représente les structures (I.a), (I.b), et (I.c) suivantes, prises ensemble ou séparément : 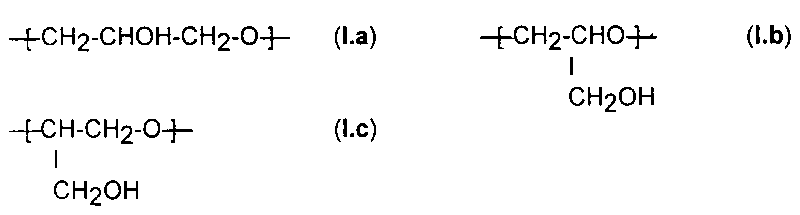 et R et n ont, ensemble, l'une des significations ci-dessous :
a) R désigne un radical ou un mélange de radicaux alkyles en C₁₀-C_{14,} et n est un nombre entier ou décimal de 2 à 10.
b) R désigne un groupement de formule **(II)**:
R₂-CO NH-CH₂-CH₂-O-CH₂-CH₂- (II),:
dans laquelle R₂ désigne un radical ou un mélange de radicaux alkyles et/ou alcényles en C₁₁-C₁₇,
et n désigne un nombre entier ou décimal de 1 à 5.
c) R désigne un groupement de formule **(III)** :
R₃-CHOH-CH₂- (III),
dans laquelle R₃ désigne un radical aliphatique, cycloaliphatique, arylaliphatique, en C₇-C₂₁, et leurs mélanges, les chaînes aliphatiques désignant en particulier des chaînes alkyles pouvant comporter de 1 à 6 groupements éther, thioéther et/ou hydroxyméthylène,
et n désigne un nombre entier ou décimal de 1 à 10.
**(B)** Les composés préparés par condensation, en catalyse acide, et à une température allant de 50 à 120°C, de 2 à 10 moles de glycidol par mole d'alcool ou d'alpha-diol comportant 10 à 14 atomes de carbone.
**(C)** Les composés préparés par polyaddition de monochlorhydrine du glycérol sur un composé organique polyhydroxylé en présence d'une base forte, avec élimination au fur et à mesure de l'eau par distillation.

9. Compositions selon la revendication 8, caractérisées par le fait que le ou les tensio-actifs non-ioniques poly(hydroxypropyléther) sont choisis parmi les composés suivants :
i) 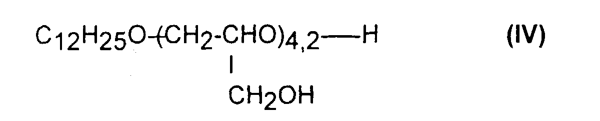 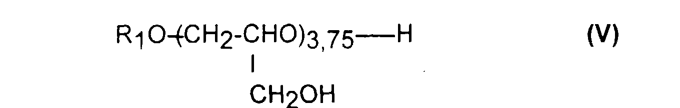 formule (V) dans laquelle R₁ désigne un mélange de radicaux alkyles en C₁₀H₂₁ et C₁₂H₂₅.
2i) les composés préparés par condensation, en catalyse alcaline, de 3,5 moles de glycidol sur un alpha-diol ayant 12 atomes de carbone.
3i) les composés de formule **(VI)** :
R₂-CONH-CH₂-CH₂-O-CH₂-CH₂-O(̵CH₂-CHOH-CH₂-O)_{3,5}-H (VI)
dans laquelle R₂ désigne un mélange de radicaux comprenant les radicaux alkyles et alcényles suivants: C₁₁H₂₃, C₁₃H₂₇, les radicaux dérivés des acides gras du coprah et le radical dérivé de l'acide oléique.
4i) les composés préparés par condensation de 3,5 moles de glycidol sur un mélange d'alpha-diols en C₁₁-C₁₄.

10. Compositions selon les revendications 8 ou 9, caractérisées par le fait que le tensio-actif non-ionique poly(hydroxypropyléther) est un dodécanediol polyglycérolé à 3,5 moles de glycérol.

11. Compositions selon l'une quelconque des revendications précédentes, caractérisées par le fait qu'il s'agit de compositions pour l'hygiène bucco-dentaire.

12. Utilisation des compositions définies à l'une quelconque des revendications précédentes, comme, ou pour la fabrication d'un, dentifrice liquide, d'une pâte dentifrice, d'un gel dentifrice ou d'une gomme à mâcher.

## Patentansprüche

1. Zusammensetzungen, die ein Abrasivmittelsystem, das ein oder mehrere Alkalihydrogencarbonate enthält, und ein Tensidsystem enthalten, das ein oder mehrere nichtionische grenzflächenaktive Poly(hydroxypropylether) enthält,
dadurch gekennzeichnet, daß
das Tensidsystem ferner mindestens ein unter Alkali- und Erdalkalilaurylsulfaten und Ammoniumlaurylsulfat ausgewähltes Tensid enthält.

2. Zusammensetzungen nach Anspruch 1, dadurch gekennzeichnet, daß das oder die Alkalihydrogencarbonate unter Natriumhydrogencarbonat und Kaliumhydrogencarbonat ausgewählt sind.

3. Zusammensetzungen nach Anspruch 1 oder 2, dadurch gekennzeichnet, daß das Laurylsulfat ein Alkalilaurylsulfat ist.

4. Zusammensetzungen nach Anspruch 3, dadurch gekennzeichnet, daß das Alkalilaurylsulfat Natriumlaurylsulfat ist.

5. Zusammensetzungen nach Anspruch 1 oder 2, dadurch gekennzeichnet, daß das Laurylsulfat ein Erdalkalilaurylsulfat ist.

6. Zusammensetzungen nach Anspruch 5, dadurch gekennzeichnet, daß das Erdalkalilaurylsulfat Magnesiumlaurylsulfat ist.

7. Zusammensetzungen nach Anspruch 1 oder 2, dadurch gekennzeichnet, daß das Laurylsulfat Ammoniumlaurylsulfat ist.

8. Zusammensetzungen nach einem der vorhergehenden Ansprüche, dadurch gekennzeichnet, daß der oder die nichtionischen grenzflächenaktiven Poly(hydroxypropylether) unter den folgenden Verbindungen ausgewählt sind:
(A) Verbindungen der Formel (I)
RO ⁅C₃H₅ (OH) O]ₙ-H (I) ,
worin die Gruppe -[C₃H₅(OH)O]- einer oder mehreren der folgenden Strukturen (Ia), (Ib) und (Ic) entspricht
⁅CH₂-CHOH-CH₂-O⁆ (Ia), 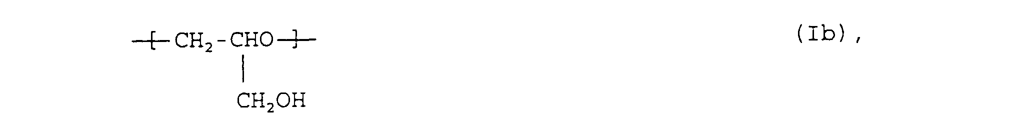 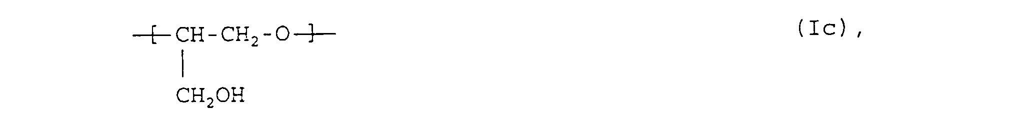
und wobei R und n jeweils in Kombination eine der folgenden Bedeutungen haben
a) R bedeutet eine C₁₀₋₁₄-Alkylgruppe oder ein Gemisch dieser Gruppen, und n ist eine ganze Zahl oder eine Dezimalzahl im Bereich von 2 bis 10.
b) R bedeutet eine Gruppe der Formel (II)
R₂-CONH-CH₂-CH₂-O-CH₂-CH₂- (II),
worin R₂ eine Alkyl- und/oder eine C₁₁₋₁₇-Alkenylgruppe bedeutet, und n ist eine ganze Zahl oder eine Dezimalzahl im Bereich von 1 bis 5.
c) R bedeutet eine Gruppe der Formel (III)
R₃-CHOH-CH₂- (III),
worin R₃ eine aliphatische, cycloaliphatische und/oder eine arylaliphatische Gruppe mit 7 bis 21 Kohlenstoffatomen bedeutet, wobei die aliphatischen Gruppen insbesondere Alkylgruppen sind, die 1 bis 6 Ether-, Thioether- und/oder Hydroxymethylengruppen aufweisen können, und n ist eine ganze Zahl oder eine Dezimalzahl im Bereich von 1 bis 10.
(B) Verbindungen, die unter saurer Katalyse bei einer Temperatur im Bereich von 50 bis 120 °C durch Kondensation von 2 bis 10 mol Glycid pro Mol eines Alkohols oder eines α-Diols mit 10 bis 14 Kohlenstoffatomen hergestellt wurden.
(C) Verbindungen, die durch Polyaddition von Glycerylmonochlorhydrin und einer organischen Polyhydroxyverbindung in Gegenwart einer starken Base hergestellt wurden, wobei das dabei gebildete Wasser durch Destillation entfernt wurde.

9. Zusammensetzungen nach Anspruch 8, dadurch gekennzeichnet, daß der oder die nichtionischen grenzflächenaktiven Poly(hydroxypropylether) unter den folgenden Verbindungen ausgewählt sind:
i) 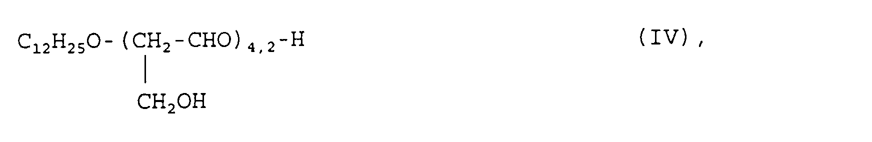 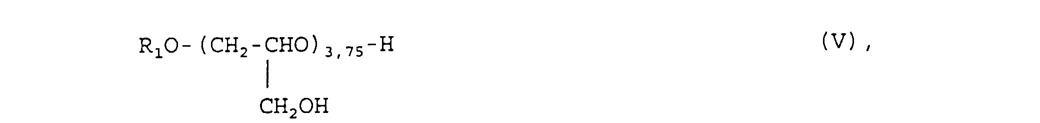 wobei in Formel (V) R₁ ein Gemisch von C₁₀H₂₁-und C₁₂H₂₅-Alkylgruppen bedeutet,
ii) Verbindungen, die unter alkalischer Katalyse durch Kondensaton von 3,5 mol Glycid und einem α-Diol mit 12 Kohlenstoffatomen hergestellt wurden,
iii) Verbindungen der Formel (VI)
R₂-CO-NH-CH₂-CH₂-O-CH₂-CH₂-O(̵CH₂-CHOH-CH₂-O)_{3,5}-H (VI),
worin R₂ ein Gemisch der folgenden Alkyl- und Alkenylgruppen bedeutet: C₁₁H₂₃, C₁₃H₂₇, Gruppen, die von Kopra-Fettsäuren stammen und der Gruppe, die von Ölsäure abgeleitet ist,
iiii) Verbindungen, die durch Kondensation von 3,5 mol Glycid und einem Gemisch von C₁₁-C₁₄-α-Diolen hergestellt wurden.

10. Zusammensetzungen nach Anspruch 8 oder 9, dadurch gekennzeichnet, daß der nichtionische grenzflächenaktive Poly(hydroxypropylether) ein mit 3,5 mol Glycerin verethertes Dodecandiol ist.

11. Zusammensetzungen nach einem der vorhergehenden Ansprüche, dadurch gekennzeichnet, daß es sich um Zusammensetzungen zur Mund- und Zahnpflege handelt.

12. Verwendung der Zusammensetzungen nach einem der vorhergehenden Ansprüche als flüssige Zahncreme, Zahnpaste, Zahngel oder Kaugummi oder zu deren Herstellung.

## Claims

1. Compositions of the type comprising an abrasive system containing one or a number of alkali metal bicarbonate(s) and a surfactant system containing one or a number of non-ionic poly(hydroxypropyl ether) surfactant(s), characterized in that the said surfactant system further comprises at least one alkali metal, alkaline-earth metal or ammonium lauryl sulphate.

2. Compositions according to Claim 1, characterized in that the alkali metal bicarbonate(s) are chosen from sodium bicarbonate or potassium bicarbonate.

3. Compositions according to Claim 1 or 2, characterized in that the lauryl sulphate is an alkali metal lauryl sulphate.

4. Compositions according to Claim 3, characterized in that the alkali metal lauryl sulphate is a sodium lauryl sulphate.

5. Compositions according to Claim 1 or 2, characterized in that the lauryl sulphate is an alkaline-earth metal lauryl sulphate.

6. Compositions according to Claim 5, characterized in that the alkaline-earth metal lauryl sulphate is a magnesium lauryl sulphate.

7. Compositions according to Claim 1 or 2, characterized in that the lauryl sulphate is an ammonium lauryl sulphate.

8. Compositions according to any one of the preceding claims, characterized in that the non-ionic poly (hydroxypropyl ether) surfactant(s) are chosen from the following compounds:
**(A)** The compounds corresponding to the formula **(I)** :
RO⁅C₃H₅ (OH)O]ₙ - H (I)
in which the ⁅C₃H₅(OH)O⁆ group represents the following structures (I.a), (I.b) and (I.c), taken together or separately: 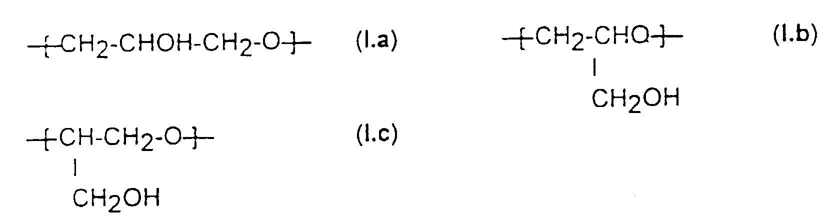 and R and n have, together, one of the meanings below:
a) R denotes a C₁₀-C₁₄ alkyl radical or a mixture of C₁₀-C₁₄ alkyl radicals, and n is a whole or decimal number from 2 to 10,
b) R denotes a group of formula **(II)** :
R₂-CONH-CH₂-CH₂-O-CH₂-CH₂- (II),
in which R₂ denotes a C₁₁-C₁₇ alkyl or alkenyl radical or a mixture of C₁₁-C₁₇ alkyl and/or alkenyl radicals,
and n denotes a whole or decimal number from 1 to 5,
c) R denotes a group of formula **(III)** :
R₃-CHOH-CH₂- (III),
in which R₃ denotes a C₇-C₂₁ aliphatic, cycloaliphatic or arylaliphatic radical, and their mixtures, the aliphatic chains denoting in particular alkyl chains which can contain from 1 to 6 ether, thioether and/or hydroxymethylene groups,
and n denotes a whole or decimal number from 1 to 10.
**(B)** The compounds prepared by condensation, in acid catalysis, and at a temperature ranging from 50 to 120°C, of 2 to 10 mol of glycidol per mole of alcohol or of alpha-diol containing 10 to 14 carbon atoms.
**(C)** The compounds prepared by polyaddition of glycerol monochlorohydrin to a polyhydroxylated organic compound in the presence of a strong base, with removal of the water, by distillation, as it is formed.

9. Compositions according to Claim 8, characterized in that the non-ionic poly(hydroxypropyl ether) surfactant(s) are chosen from the following compounds:
i) 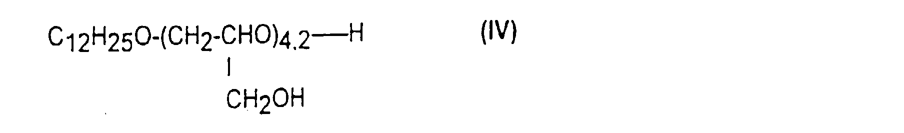 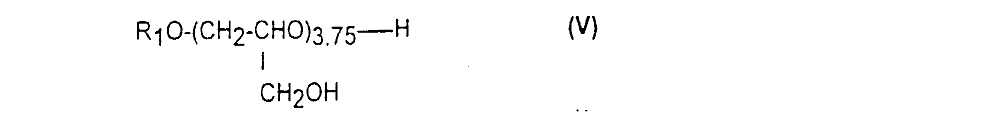 in which formula (V) R₁ denotes a mixture of C₁₀H₂₁ and C₁₂H₂₅ alkyl radicals,
2i) the compounds prepared by condensation, in alkaline, catalysis, of 3.5 mol of glycidol with an alpha-diol having 12 carbon atoms,
3i) the compounds of formula (VI):
R₂-CONH-CH₂-CH₂-O-CH₂-CH₂-O-(CH₂-CHOH-CH₂-O)_{3.5}-H (VI)
in which R₂ denotes a mixture of radicals comprising the following alkyl and alkenyl radicals: C₁₁H₂₃, C₁₃H₂₇, the radicals derived from coconut fatty acids and the radical derived from oleic acid,
4i) the compounds prepared by condensation of 3.5 mol of glycidol with a mixture of C₁₁-C₁₄ alpha-diols.

10. Compositions according to Claim 8 or 9, characterized in that the non-ionic poly(hydroxypropyl ether) surfactant is a dodecanediol polyglycerolated with 3.5 mol of glycerol.

11. Compositions according to any one of the preceding claims, characterized in that they are compositions for oral hygiene.

12. Use of the compositions defined in any one of the preceding claims as, or for the manufacture of, a liquid dentrifice, a toothpaste, a tooth gel or a chewing gum.
